# EUROPÄISCHE PATENTSCHRIFT

(11) **EP 2 846 738 B1**
(45) Veröffentlichungstag und Bekanntmachung des Hinweises auf die Patenterteilung: **20.11.2019**
(21) Anmeldenummer: 13721739.4
(22) Anmeldetag: 08.05.2013
(51) Int. Cl.: A61F 2/30, A61B 17/72, A61F 2/36, A61F 2/38

(54) **VERSTÄRKUNGSIMPLANTAT FÜR EINEN LANGGESTRECKTEN KNOCHEN, INSBESONDERE FEMUR**
REINFORCEMENT IMPLANT FOR A LONG BONE, IN PARTICULAR THE FEMUR
IMPLANT DE RENFORCEMENT POUR UN OS ALLONGÉ, EN PARTICULIER LE FÉMUR

(30) Priorität: 11.05.2012 EP 12167795
(43) Veröffentlichungstag der Anmeldung: 18.03.2015
(73) Patentinhaber: Waldemar Link GmbH & Co. KG, 22339 Hamburg (DE)
(72) Erfinder: LINK, Helmut D., 22397 Hamburg (DE)
(74) Vertreter: Glawe, Delfs, Moll
(86) Internationale Anmeldenummer: PCT/EP2013/059589
(87) Internationale Veröffentlichungsnummer: WO 2013/167655

(56) Entgegenhaltungen:
- WO-A1-00/30569
- US-A- 5 062 849
- US-B1- 7 998 218

## Beschreibung

Die Erfindung betrifft ein Verstärkungsimplantat für Verankerungsschäfte zweier gegenüberliegend an einem langgestreckten Knochen, insbesondere Femur, angeordneten Prothesen.

Am menschlichen Körper sind im Bereich der großen Extremitäten häufig Prothesen, insbesondere Gelenkprothesen, erforderlich aufgrund von Verschleiß oder Fehlbildung. Von besonderer praktischer Relevanz ist hierbei der Femur, also der Oberschenkelknochen. Sein oberes Ende wirkt mit dem Hüftgelenk zusammen, während sein unteres Ende mit dem Kniegelenk des Menschen zusammenwirkt. Hüftoperationen mit der Implantation eines künstlichen Hüftgelenks kommen verhältnismäßig häufig vor. Dann befindet sich am oberen Ende des Femurs der femurseitige Teil einer Hüftgelenksendoprothese, der einen mittels eines Verankerungsschafts befestigten Kugelkopf zum gelenkigen Zusammenwirken mit einem im Becken angeordneten Gegenstück aufweist. Der den Kugelkopf tragende Verankerungsschaft erstreckt sich in den Markkanal des Femurs, und kann ja nach Prothesenbauart und Größe sich bis in eine beträchtliche Tiefe des Markkanals erstrecken. In Bezug auf das Kniegelenk gilt ähnliches, es ist ebenso recht häufig Gegenstand einer Operation, bei der ein künstliches Kniegelenk eingesetzt wird. Wie auch bei der Hüftgelenksendoprothese, wird bei der Kniegelenksendoprothese der femurseitige Teil der Prothese über einen Verankerungsschaft gesichert, der von unten in den Markkanal des Femurs eingeschoben ist. In der Praxis ist es gar nicht so selten, dass bei einem Patienten an einem Femur beide Gelenkendoprothesen implantiert sind. Dies braucht nicht unbedingt zeitgleich geschehen zu sein, häufig geschieht dies auch zeitlich hintereinander.

Es hat sich in der Praxis gezeigt, dass zwar jede der beiden Gelenkendoprothesen für sich genommen eine gute Funktionalität wie auch Dauerhaltbarkeit aufweist. Jedoch, und das ist in einigen Fällen entscheidend, kann es bei dem Vorhandensein von zwei solchen Gelenkendoprothesen an einem Femur zu einer negativen Interaktion kommen. Diese besteht insbesondere darin, dass die von den beiden Enden in den Femur eingesteckten Verankerungsschäfte eine Verstärkung des Femurs in den jeweiligen Endbereichen bewirken, in denen sich die Verankerungsschäfte im Markkanal des Femurs erstrecken. Nicht verstärkt ist hingegen der Zwischenbereich, in dem sich keiner der beiden Verankerungsschäfte erstreckt. Damit wird dieser, obgleich biologisch an sich gesund, relativ geschwächt verglichen mit den durch den Verankerungsschaft verstärkten Endbereichen, so dass es hier wegen der ungleichmäßigen Kraftverteilung und somit besonders hohen Belastung nicht selten zu Knochenfrakturen kommt.

Es ist bekannt, dass zur Verstärkung des Knochens in dem Zwischenbereich Knochenplatten vorgesehen sein können. Sie bieten den Vorteil, dass ihre Implantation praktisch gut beherrscht wird und dass sie im Übrigen auch nachträglich gut zu implantieren sind. Letzteres ist insbesondere in dem Fall von Bedeutung, wenn die Versorgung mit Gelenkprothesen zeitlich versetzt geschieht. In diesem Fall braucht die verstärkende Knochenplatte erst dann implantiert zu werden, wenn die zweite Gelenkendoprothese (beispielsweise die Knieprothese bei bereits vorhandener Hüftgelenksprothese) implantiert wird. Ein Nachteil der Knochenplatten besteht darin, dass sie zur Erreichung einer ausreichenden mechanischen Stabilität verhältnismäßig groß sein müssen, was eine entsprechend beträchtlich große Operationswunde im Zwischenbereich des Femurs erfordert. Ein weiterer Nachteil, der sich im praktischen Einsatz gezeigt hat, ist dass die Stabilität der Verstärkung häufig unbefriedigend ist. Insbesondere kommt es zu einem Ausreißen der zur Befestigung der Knochenplatten verwendeten Schrauben oder gar zu einem Bruch der Knochenplatte. Eine Verstärkung der Schraubbefestigung ist schwierig, da die Anordnungsmöglichkeiten begrenzt sind aufgrund der im Verankerungsbereich der Knochenschrauben bereits liegenden Verankerungsschäfte. Bezüglich der Platten zeigt es sich, dass eine Verstärkung ebenfalls schwierig ist, da nur eine begrenzte Weichteildeckung in diesem Bereich vorliegt. Der Gefahr eines Versagens der mit den Knochenplatten bewirkten Verstärkung wird also nicht wirksam begegnet, was den ohnehin bereits durch zwei schwere Gelenkendoprothesen-Operationen belasteten Patienten zusätzliche Risiken aufbürdet.

Aus der WO 00/30569 A1 ist eine Armierungsendoprothese bekannt, die an ihren beiden Enden Aufnahmemuffen für je einen Verankerungskonus einer Gelenkendoprothese aufweist. Dazwischen ist ein zweiteiliger Marknagel als Kopplungsstück vorgesehen. Die beiden Komponenten des zweiteiligen Marknagels sind über ein Gewinde miteinander verbunden, sodass durch gegenseitiges Verdrehen die Länge der Endoprothese einstellbar ist. Damit ist die Armierungsendoprothese genau auf den vorgesehenen Anwendungsfall in der Länge abgestimmt. Eine nachträgliche Einstellung im implantierten Zustand ist nicht vorgesehen, sondern erfolgt zusammen mit der Implantation der beiden Gelenkendoprothesen an den beiden Enden der Armierungsendoprothese. Diese Armierungsendoprothese ist also genau angepasst an den vorgesehenen Anwendungsfall. Sollte einer der beiden Gelenkendoprothesen erst am späteren Zeitpunkt implantiert werden, so muss ihr Verbindungskonus genau hinsichtlich Länge und Durchmesser zu der bereits implantierten Armierungsendoprothese passen. Damit ist sie nicht geeignet zur Versorgung in solchen Fällen, bei denen unterschiedliche Gelenkendoprothesen zum Einsatz kommen, oder bei denen einer der beiden Gelenkendoprothesen erst zu einem späteren Zeitpunkt implantiert wird.

Aus der US 7,998,218 B1 ist ein modulares Prothesensystem bekannt, bei welchem der Schaft durch verschiedene Einsätze mit unterschiedlichen Längen gebildet ist. Eine Längeneinstellung kann so nur im Rahmen der Erstimplantation erfolgen. Das System eignet sich daher nicht für solche Fälle, bei denen eine der beiden Prothesen erst zu einem späteren Zeitpunkt implantiert wird.

Aus der US 5,062,849 A1 ist ein Verstärkungsimplantat bekannt, das als eine Hülle zur Aufnahme von zwei Verankerungsschäften ausgebildet ist. Die kraftschlüssige Verbindung erfolgt durch die freien Enden der Verankerungsschäfte, die zu diesem Zweck gegensinnig keilförmig angeschrägt sind.

Der Erfindung liegt die Aufgabe zugrunde, ein Verstärkungsimplantat der eingangs genannten Art zu schaffen, welches diese Nachteile vermeidet.

Die erfindungsgemäße Lösung liegt in einem Verstärkungsimplantat mit den Merkmalen des Anspruchs 1. Vorteilhafte Weiterbildungen sind Gegenstand der abhängigen Ansprüche. Bei einem Verstärkungsimplantat für Verankerungsschäfte zweier gegenüberliegend an einem langgestreckten Knochen, insbesondere Femur, angeordneten Prothesen ist erfindungsgemäß vorgesehen, dass das Verstärkungsimplantat eine generell langgestreckte schaftartige Form aufweist und an seinen beiden Enden je eine Aufnahmemuffe für jeweils einen der Verankerungsschäfte und dazwischen liegend ein trennbares Kopplungsstück zur starren Verbindung der beiden Aufnahmemuffen aufweist.

Die Erfindung beruht auf dem Gedanken, dass mit den Aufnahmemuffen die beiden Verankerungsschäfte fest ergriffen werden und durch das an dem Verstärkungsimplantat vorgesehene Kopplungsstück kraftschlüssig miteinander verbunden werden. Dadurch wird eine Kraftbrücke zwischen den beiden Verankerungsschäften gebildet, so dass der Knochen insbesondere in dem empfindlichen Zwischenbereich zwischen den beiden Verankerungsschäften nicht mehr mit der Kraftübertragung belastet wird. Es ergibt sich damit eine Schonung des Knochens. Weiter ergibt sich eine gleichmäßigere Kraftverteilung im Knochen, da dieser nun über die gesamte Länge seines Femurkanals mit einer Verstärkung versehen ist. Verwerfungen in Bezug auf die Stabilität des Knochens werden damit wirkungsvoll vermieden. Damit wird erreicht, dass keine Brüche mehr im Zwischenbereich auftreten. Die Aufnahmemuffen sind als Universalaufnahmen für Verstärkungsschäfte mit unterschiedlichen Dimensionen ausgebildet. Dies ermöglicht es, unterschiedliche Arten wie auch Größen von Gelenkendoprothesen mit ihren Verstärkungsschäften aufzunehmen und sicher zu verankern. Dies ist insbesondere deshalb von Bedeutung, da gerade bei der zeitversetzten Implantation es nicht ausgeschlossen werden kann, dass die Gelenkendoprothese an dem einem Ende von einem anderen Hersteller als die Gelenkendoprothese an dem anderen Ende stammt, so dass die Verankerungsschäfte vollkommen unterschiedlich ausgebildet sind. Mit der universellen Aufnahme in der Muffe wird auch für diesen Fall eine sichere und zuverlässige Befestigung erreicht. Dazu ist die Aufnahmemuffe so ausgebildet, dass eine in ihr angeordnete Aufnahmebohrung bezüglich ihrer Weite abgestuft ist. Das bedeutet, dass der äußere Bereich der Aufnahmebohrung eine größere Weite aufweist, als ein innenliegender, d.h. zum Kopplungsstück weisender Bereich der Aufnahmebohrung. Mit einer solchen gestuften Ausnahmebohrung können nicht nur Schäfte unterschiedlicher Weite bzw. unterschiedlichen Durchmessers sicher aufgenommen werden, sondern es ermöglicht auch eine besonders sichere Halterung von Verankerungsschäften mit konischen Endbereichen.

Um die Verankerungsschäfte in der Aufnahmemuffe zu arretieren und ein unbeabsichtigtes Herauswandern aus der Ausnahmemuffe zu vermeiden, sind vorzugsweise mehrere Befestigungslöcher reihenweise an der Aufnahmemuffe angeordnet. Die Befestigungslöcher sind zur Aufnahme von Befestigungsschrauben ausgebildet. Dadurch kann der Verankerungsschaft fest in die Aufnahmemuffe eingespannt sein. Damit wird auch bei großen Belastungen oder über längere Zeiträume von mehreren Jahren hinweg eine ausreichend starke und haltbare Befestigung realisiert. Vorzugsweise sind die Befestigungslöcher über den Umfang axial versetzt angeordnet. Dies ermöglicht eine sichere Befestigung auch von nichtrotationssymmetrisch geformten Verankerungsschäften.

Zur Gewährleistung einer sicheren Verankerung sind die Befestigungsschrauben vorzugsweise mit kegeligen Spitzen versehen. Dies sorgt durch hohe Flächenpressung auf den Außenmantel des Verankerungsschafts für eine sichere und wackelfreie Befestigung. Besonders bevorzugt ist es, wenn die Spitze aus einem Hartmetall besteht, welches aufgrund seiner Härte befähigt ist zum Eindringen in den Außenmantel des Schafts. Auf diese Weise wird eine noch sicherere Befestigung erreicht.

Mit Vorteil sind die Befestigungslöcher mit Sicherungseinrichtungen versehen, die ein unbeabsichtigtes Lösen der Befestigungsschrauben verhindern. Damit wird eine hohe Befestigungssicherheit auch über lange Zeiträume von Jahren oder Jahrzehnten erreicht. Die Sicherungseinrichtungen können in Form von Kunststoffeinsätzen ausgeführt sein.

Die Aufnahmemuffe ist zweckmäßigerweise so lang bemessen, dass ihre Länge mindestens das dreifache der Weite des Verankerungsschafts beträgt. Es hat sich gezeigt, dass mit einer solchen, verhältnismäßig langen Abstützung des Verankerungsschafts eine bessere und stabilere Verbindung im Femur erreicht werden kann. Gerade bei dem Femur ist dies von besonderer Bedeutung, da es aufgrund seiner Länge zu großen Hebelkräften an den beiden beteiligten Gelenkendoprothesen, Hüfte bzw. Knie, kommen kann.

Zweckmäßigerweise ist das Koppelstück hingegen kurz ausgeführt. Unter kurz wird vorliegend verstanden, dass seine Länge nicht größer ist als die Weite der Aufnahmemuffe. Damit wird gerade in dem hochbelasteten Bereich des Koppelstücks durch Verkürzung der Hebelarme eine geringere Belastung erreicht. Die Stabilität des erfindungsgemäßen Verstärkungsimplantats steigt dadurch.

Mit Vorteil ist das Koppelstück als ein Keilverbinder ausgeführt. Dieser bietet den Vorteil, eine spielfreie Befestigung der beiden Komponenten, also der beiden Aufnahmemuffen, auch bei hoher Kraftbeaufschlagung zu ermöglichen.

Der Keilverbinder ist vorzugsweise so ausgeführt, dass er zwei gegensinnig angeordnete Flachkeile umfasst. Unter gegensinnig angeordnet wird verstanden, dass jeder der beiden Flachkeile an einem der beiden Aufnahmemuffen angeordnet ist und sich in Richtung des anderen erstreckt. Unter Flachkeile werden Keile mit an dem im wesentlichen rechteckförmigen Querschnitt verstanden, wobei die Höhe des Rechtecks je nach Position am Keil zur Spitze hin kontinuierlich abnimmt. Damit wird eine besonders kompakte und belastbare Verbindung im Kopplungsstück realisiert.

Mit Vorteil sind Sicherungsschrauben für den Keilverbinder vorgesehen. Sie ermöglichen eine spielfreie Fixierung des Keilverbinders bereits bei der Operation, ermöglichen aber auch ggf. ein Lösen der Verbindung im Fall einer erforderlich werdenden Reimplantation. Vorzugsweise sind die Sicherungsschrauben so implantiert, dass sie mit mindestens einer Reihe von Befestigungslöchern an der Aufnahmemuffe fluchten. Dies erleichtert den Zugang zu den Schrauben während der Operation.

Die Erfindung wird nachfolgend unter Bezugnahme auf die beigefügte Zeichnung, in der, ein vorteilhaftes Ausführungsbeispiel dargestellt ist, näher erläutert. Es zeigen:
- Fig. 1:: eine perspektivische Ansicht eines Ausführungsbeispiels für ein Verstärkungsimplantat;
- Fig. 2 a, b:: eine Aufsicht sowie eine Seitenansicht des in Figur 1 dargestellten Ausführungsbeispiels;
- Fig. 3:: eine Darstellung der Einzelteile des Verstärkungsimplantats gemäß dem ersten Ausführungsbeispiel;
- Fig. 4:: das Verstärkungsimplantat im implantierten Zustand an einem Femur in einer Röntgendarstellung;
- Fig. 5:: ein Bruch des Femurs ohne Verstärkungsimplantat in einer Röntgendarstellung.

Ein Ausführungsbeispiel für ein erfindungsgemäßes Verstärkungsimplantat wird nachfolgend beschrieben. Es umfasst als Hauptkomponenten jeweils eine Aufnahmemuffe 1, 2 an sein beiden Enden sowie dazwischen ein Kopplungsstück 3. Die Aufnahmemuffe 1 ist auf der distalen Seite, d.h. an der dem Ende einer Extremität des Körpers zugewandten Seite, während die Aufnahmemuffe 2 auf der proximalen Seite angeordnet ist.

Nachfolgend wird der Aufbau der beiden Aufnahmemuffen 1, 2 beschrieben. Es geschieht am Beispiel der distalen Aufnahmemuffe 1. Die proximale Aufnahmemuffe 2 ist identisch aufgebaut, soweit darauf nicht gesondert eingegangen wird. Die distale Aufnahmemuffe 1 ist von im wesentlichen hohlzylindrischer Gestalt mit einem glatten Außenmantel. Es ist vorzugsweise aus einem biokompatiblen Metallmaterial, insbesondere Kobaltchrommolybden (CoCrMo) hergestellt. Sein Außendurchmesser ist so gewählt, dass er nicht größer ist als die Weite des Knochens, an dem die Implantation vorgesehen ist. In dem dargestellten Ausführungsbeispiel ist das Verstärkungsimplantat zur Implantation an einem Oberschenkelknochen (Femur 9) vorgesehen.

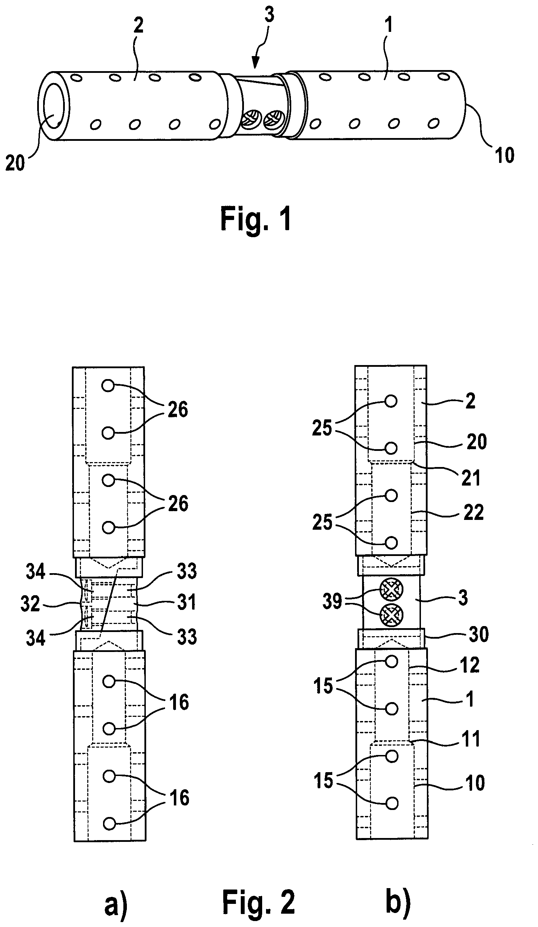

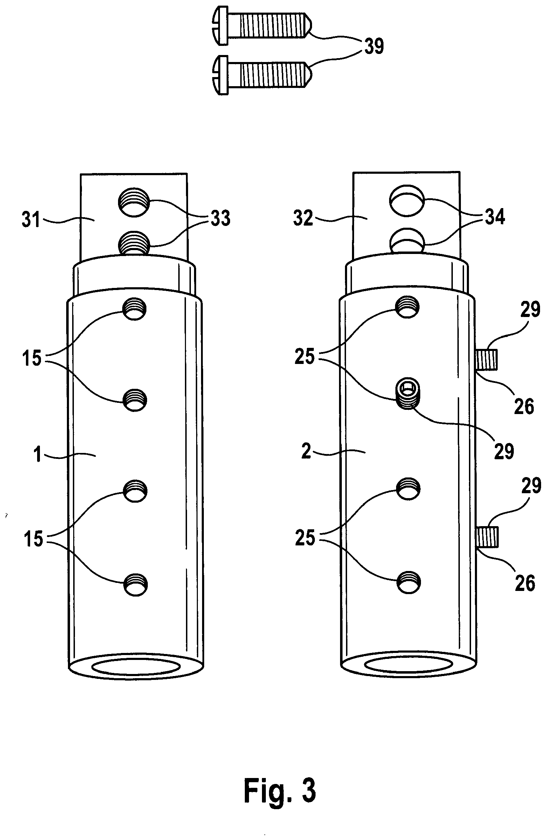

Die Aufnahmemuffe 1 weist in ihrem Innenraum einen in Axialrichtung verlaufenden Hohlraum 10 auf. Dieser ist gestuft ausgeführt mit einer etwa auf halber Tiefe befindlichen Schulter 11, an der sich ein Bereich mit verengtem Durchmesser 12 anschließt. Der Innenraum 10 mündet an einer Stirnseite der Aufnahmemuffe 1. Im Mantel der Aufnahmemuffe 1 sind jeweils gegenüberliegend Paare von Lochreihen ausgebildet. An einer Vorder- bzw. Rückseite sind vier Löcher 15 angeordnet (siehe Figur 2b), während in Axialrichtung versetzt an den beiden Lateralseiten ebenfalls vier Löcher 16 (siehe Figur 2a) angeordnet sind. Der Versatz ist dabei so gewählt, dass eines der Löcher der Lochreihen mit den Löchern 16 etwa in der Mitte zwischen den Löchern der Lochreihe mit den Löchern 15 angeordnet ist und umgekehrt. Dies ist aus Figur 1 gut zu erkennen. Die Aufnahmelöcher sind vorzugsweise mit einem Gewinde versehen zur Aufnahme von einer Befestigungsschraube 29 (siehe Figur 3). Die Aufnahmelöcher 15, 16 sind so ausgerichtet, dass sie zur Mittelachse des Innenraums 10 zeigen. Sie dienen zur Befestigung eines in den Aufnahmeraum eingeschobenen Verankerungsschafts, wie später noch näher erläutert werden wird.

An dem der Stirnseite gegenüberliegenden Ende der proximalen Aufnahmemuffe 1 ist ein Keilelement 31 des Koppelstücks 3 angeordnet. Es wirkt zusammen mit einem komplementär aufgebauten Keilelement 32 an der distalen Aufnahmemuffe 2. Die Keilelemente 31, 32 sind so angeordnet, dass sie mit ihrer Schrägfläche aufeinander zu liegen kommen. Das Keilelement 32 weist zwei in Axialrichtung der Aufnahmemuffe 2 hintereinander gelegene Aufnahmebohrungen 34 auf, die im zusammengesetzten Zustand mit zwei entsprechend in Axialrichtung hintereinander angeordneten Aufnahmebohrungen 33 an der Aufnahmemuffe 1 fluchten. In den Aufnahmebohrungen 33 ist ein Innengewinde ausgebildet. In die fluchtenden Bohrungen 34, 33 wird im zusammengesetzten Zustand jeweils eine Sicherungsschraube 39 eingeschraubt, welche in das Innengewinde der Aufnahmeöffnung 33 eingreift und somit festgezogen wird. Auf diese Weise wird erreicht, dass die beiden Keilelemente 31, 32 des Koppelstücks fest gegeneinander gezogen sind und damit eine kraft- wie auch formschlüssige Verbindung bilden. Diese Verbindung kann außerordentlich hohe Kräfte aufnehmen und ist im übrigen dank der Keilwirkung spielfrei.

Als Beispiel für Abmessungen des Ausführungsbeispiels wird für die Aufnahmemuffen 1, 2 eine Länge von ca. 90 mm gewählt und für das Koppelstück eine Länge von ca. 25 mm. Die Weite des Innenraums 10 ist im Bereich der Stirnseite etwa 18 mm und beträgt im tieferen, verengten Bereich etwa 15 mm. Der Gesamtdurchmesser der Aufnahmemuffe beträgt etwa 30 mm. Die Keilelemente 31, 32 weisen vorzugsweise eine Weite und im zusammengesetzten Zustand auch eine Stärke von etwa 25 mm auf. In dem dargestellten Ausführungsbeispiel ist eine zweckmäßige Variante abgebildet, nämlich bei der die Weite des Aufnahmeraums in der proximalen Aufnahmemuffe 2, d.h. in dem Innenraum 20 etwas erhöht ist verglichen mit derjenigen des Innenraums 10 an der distalen Aufnahmemuffe 1. Dies hat den Effekt, dass damit der in der Regel größere Schaft 96 einer Hüftprothese 94 nur in den Innenraum der proximalen Aufnahmemuffe 2 eingeschoben werden kann, und nicht umgekehrt. Damit wird zum einen eine bessere Halterung erreicht, und zum anderen eine Verwechselungssicherheit bewirkt.

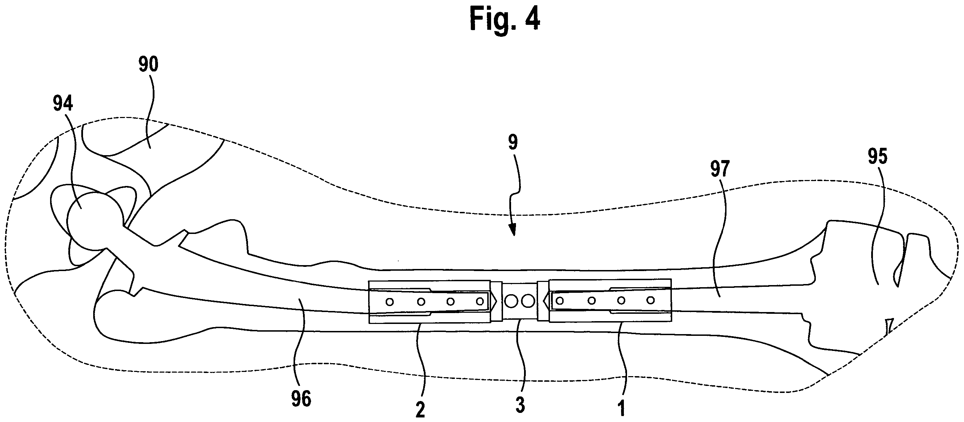

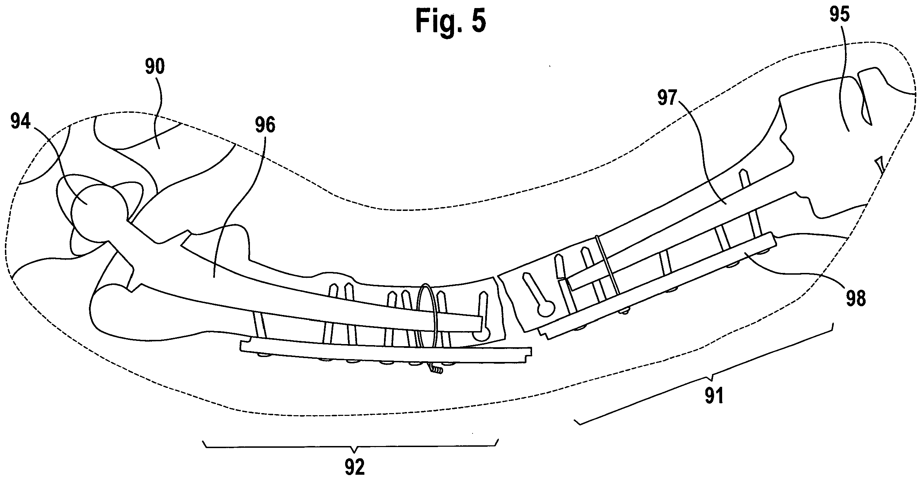

Die Anwendung des Ausführungsbeispiels für das erfindungsgemäße Verstärkungsimplantat wird in Bezugnahme auf die Figuren 4 und 5 erläutert. Ausgangspunkt sei die in Figur 5 dargestellte Situation, bei welcher eine Fraktur 9 eines Femurs 9 vorliegt. Der Femur 9 ist vorsorgt mit einem künstlichen Hüftgelenk 94, welches über einen Verankerungsschaft 96 in einem proximalen Teil 92 des Femurs 9 implantiert ist. Am gegenüberliegenden, distalen Ende des Femurs ist eine Kniegelenkendoprothese 95 implantiert, die über ihren Verankerungsschaft 97 in einem distalen Bereich 91 des Femurs befestigt ist. Deutlich erkennt man in der Röntgenaufnahme gemäß Figur 5 die beiden Verankerungsschäfte 96, 97, und wie weit sie in den Markkanal des Femurs 9 ragen, bis sie sich beinahe berühren. Daraus ist weiter zu erkennen, dass die proximale distalen Bereiche 92, 91 durch den in diesem Bereich eingeschobenen Verankerungsschaft 96 bzw. 97 jeweils verstärkt sind, der dazwischen liegende Bereich aber nicht. Damit ergibt sich die eingangs erwähnte Problematik einer ungleichmäßigen Belastung des Femurs 9. Im Stand der Technik ist dazu versucht worden, eine Knochenplatte 98 außen an dem Femur zu befestigen, um so den Zwischenbereich zu verstärken. Wie man erkennt, ist diese Maßnahme in der Praxis häufig nicht ausreichend, da es zu einem Bruch der Knochenplatte 98 und einem Ausreißen ihrer Befestigungsschrauben kommt.

Die erforderliche Befestigungssicherheit kann mit dem erfindungsgemäßen Verstärkungsimplantat erreicht werden. Das Verstärkungsimplantat wird mit seinem einem Teil, vorzugsweise dem distalen Teil umfassend die distale Aufnahmemuffe 1 auf das freie Ende des Verankerungsschafts 97 der Kniegelenkendoprothese 95 aufgeschoben. Das Keilelement 31 weist zur Mitte des Femurs 9 hin, in dem Fall also nach proximal. Im Gegenzug wird dann auf den Verankerungsschaft 96 der Hüftgelenkendoprothese 94 die proximale Aufnahmemuffe 2 aufgeschoben, wobei deren Keilelement 32 ebenfalls zur Mitte des Femurs zeigt, in dem Fall nach distal. Beiden Komponenten werden Befestigungsschrauben 29 in die jeweiligen Befestigungslöcher 15, 16 sowie 25 und 26 eingeschraubt, bis die Verankerungsschäfte 96, 97 jeweils fixiert sind. Die Befestigung mittels der Schrauben ermöglicht hierbei eine Variabilität in Bezug auf die Achse, d.h. die Verankerungsschäfte 96, 97 können Achsabweichungen aufweisen und werden dennoch in den Elementen des Verstärkungsimplantats festgehalten. Beide Elemente werden von ihrem jeweiligen Ende aus in den Markkanal des Femurs 9 eingeschoben, bis die Keilelemente 31, 32 in Anlage zueinander kommen (wie in Figur 1 dargestellt). In dieser Situation ist das Koppelelement 3 zusammengefügt, so dass die Sicherungsschrauben 39 in die Öffnungen 33, 34 eingeschraubt und festgezogen werden können. Damit ist das Implantat fixiert. Es ergibt sich eine durchgehende Verbindung, die dank dieser Kopplung stabil ist. Brüche, wie in Figur 5 dargestellt, werden damit zuverlässig vermieden.

## Patentansprüche

1. Verstärkungsimplantat für Verankerungsschäfte (96, 97) zweier gegenüberliegend an einem langgestreckten Knochen, insbesondere Femur (9) angeordneten Prothesen (94, 95), das eine generell langgestreckte schaftartige Form aufweist und an seinen beiden Enden je eine Aufnahmemuffe (1, 2) für je einen der Verankerungsschäfte (96, 97) und dazwischen liegend ein trennbares Kopplungsstück (3) zur starren Verbindung aufweist, wobei die Aufnahmemuffen (1, 2) als Universalaufnahmen für die Verankerungsschäfte (96, 97) mit unterschiedlichen Dimensionen ausgebildet sind,
**dadurch gekennzeichnet, dass**
an den Aufnahmemuffen (1, 2) mehrere Befestigungslöcher (15, 16, 25, 26) reihenweise angeordnet sind, und die Befestigungslöcher (15, 16, 25, 26) über den Umfang axial versetzt angeordnet sind, wobei die Befestigungslöcher (15, 16, 25, 26) zur Aufnahme von Befestigungsschrauben (29) ausgebildet sind und wobei die Aufnahmebohrungen (10, 20) mit den Befestigungslöchern (15, 16, 25, 26) so zusammenwirken, dass eine polyaxiale Aufnahme der Verankerungsschäfte (96, 97) gebildet ist, vorzugsweise mit einer Winkelabweichung von bis zu 10° zur Achse der Aufnahmemuffe (1, 2).

2. Verstärkungsimplantat nach Anspruch 1,
**dadurch gekennzeichnet, dass**
die Aufnahmemuffen (1, 2) als Universalaufnahmen für die Verankerungsschäfte (96, 97) mit unterschiedlichen Dimensionen ausgebildet sind.

3. Verstärkungsimplantat nach Anspruch 2,
**dadurch gekennzeichnet, dass**
eine Aufnahmebohrung (10, 20) in den Aufnahmemuffen (1, 2) vorgesehen ist, deren Weite abgestuft ist.

4. Verstärkungsimplantat nach einem der Ansprüche 1 bis 3,
**dadurch gekennzeichnet, dass**
die Befestigungsschrauben (29) kegelige Spitzen aufweisen, vorzugsweise aus Hartmetall.

5. Verstärkungsimplantat nach einem der Ansprüche 1 bis 4,
**dadurch gekennzeichnet, dass**
die Befestigungslöcher (15, 16, 25, 26) mit Sicherungseinrichtungen versehen sind, die ein unbeabsichtigtes Lösen verhindern.

6. Verstärkungsimplantat nach einem der vorangehenden Ansprüche,
**dadurch gekennzeichnet, dass**
die Aufnahmemuffen (1, 2) eine Länge von mindestens dem dreifachen der Weite des Verankerungsschafts (96, 97) aufweisen.

7. Verstärkungsimplantat nach einem der vorangehenden Ansprüche,
**dadurch gekennzeichnet, dass**
das Koppelstück (3) kurz ist, d.h. eine Länge von weniger als die Weite der Aufnahmemuffe (1, 2) aufweist.

8. Verstärkungsimplantat nach einem der vorangehenden Ansprüche,
**dadurch gekennzeichnet, dass**
das Koppelstück (3) als Keilverbinder ausgeführt ist.

9. Verstärkungsimplantat nach Anspruch 8,
**dadurch gekennzeichnet, dass**
der Keilverbinder gegensinnig angeordnete Flachkeile (31, 32) umfasst.

10. Verstärkungsimplantat nach Anspruch 8 oder 9,
**dadurch gekennzeichnet, dass**
Sicherungsschrauben (39) für den Keilverbinder vorgesehen sind.

11. Verstärkungsimplantat nach Anspruch 10,
**dadurch gekennzeichnet, dass**
die Sicherungsschrauben (39) so orientiert sind, dass sie in Linie mit mindestens einer Reihe von Befestigungslöchern (15, 16) an der Aufnahmemuffe (1, 2) stehen.

## Claims

1. Reinforcement implant for anchoring shafts (96, 97) of two prostheses (94, 95) which are arranged opposite each other on a long bone, in particular a femur (9), said reinforcement implant being of a generally elongate shaft-like shape and having a receiving sleeve (1, 2) at both ends for a respective anchoring shaft (96, 97) and, lying between said receiving sleeves (1, 2), a disconnectable coupling piece (3) for rigid connection, the receiving sleeves (1, 2) being configured as universal sockets for the anchoring shafts (96, 97) with different dimensions, **characterized in that** a plurality of fastening holes (15, 16, 25, 26) are arranged in rows on the receiving sleeves (1, 2), and the fastening holes (15, 16, 25, 26) are arranged axially offset about the circumference, wherein the fastening holes (15, 16, 25, 26) are configured to receive fastening screws (29), and wherein the receiving bores (10, 20) interact with the fastening holes (15, 16, 25, 26) such that a polyaxial seat for the anchoring shafts (96, 97) is formed, preferably with an angle deviation of up to 10° to the axis of the receiving sleeve (1, 2).

2. Reinforcement implant according to Claim 1, **characterized in that** the receiving sleeves (1, 2) are configured as universal sockets for the anchoring shafts (96, 97) with different dimensions.

3. Reinforcement implant according to Claim 2, **characterized in that** a receiving bore (10, 20) is provided in the receiving sleeves (1, 2), the width of which receiving bore (10, 20) is stepped.

4. Reinforcement implant according to one of Claims 1 to 3, **characterized in that** the fastening screws (29) have conical tips, preferably made of hard metal.

5. Reinforcement implant according to one of Claims 1 to 4, **characterized in that** the fastening holes (15, 16, 25, 26) are provided with securing devices which prevent accidental loosening.

6. Reinforcement implant according to one of the preceding claims, **characterized in that** the receiving sleeves (1, 2) have a length that is at least three times the width of the anchoring shafts (96, 97).

7. Reinforcement implant according to one of the preceding claims, **characterized in that** the coupling piece (3) is short, i.e. has a length that is less than the width of the receiving sleeve (1, 2).

8. Reinforcement implant according to one of the preceding claims, **characterized in that** the coupling piece (3) is designed as a wedge action connector.

9. Reinforcement implant according to Claim 8, **characterized in that** the wedge action connector comprises flat wedges (31, 32) arranged in opposite directions.

10. Reinforcement implant according to Claim 8 or 9, **characterized in that** locking screws (39) are provided for the wedge action connector.

11. Reinforcement implant according to Claim 10, **characterized in that** the locking screws (39) are oriented such that they are in line with at least one row of fastening holes (15, 16) on the receiving sleeve (1, 2).

## Revendications

1. Implant de renforcement pour tiges d'ancrage (96, 97) de deux prothèses (94, 95) disposées au niveau d'un os allongé, notamment un fémur (9), lequel possède une forme de type tige globalement allongée et possède à ses deux extrémités respectivement un manchon d'accueil (1, 2) pour respectivement l'une des tiges d'ancrage (96, 97) et, interposée entre les deux, une pièce de couplage (3) séparable destinée à une liaison rigide, les manchons d'accueil (1, 2) étant réalisés sous la forme de logements universels pour les tiges d'ancrage (96, 97) ayant des dimensions différentes, **caractérisé en ce que**
plusieurs trous de fixation (15, 16, 25, 26) sont disposés en rangée au niveau des manchons d'accueil (1, 2), et les trous de fixation (15, 16, 25, 26) sont disposés décalés axialement sur le pourtour, les trous de fixation (15, 16, 25, 26) étant configurés pour accueillir des vis de fixation (29) et les alésages d'accueil (10, 20) coopérant avec les trous de fixation (15, 16, 25, 26) de telle sorte qu'un accueil polyaxial des tiges d'ancrage (96, 97) est formé, de préférence avec un écart angulaire maximal de 10° par rapport à l'axe du manchon d'accueil (1, 2).

2. Implant de renforcement selon la revendication 1, **caractérisé en ce que** les manchons d'accueil (1, 2) sont réalisés sous la forme de logements universels pour les tiges d'ancrage (96, 97) ayant des dimensions différentes.

3. Implant de renforcement selon la revendication 2, **caractérisé en ce qu'**un alésage d'accueil (10, 20) se trouve dans les manchons d'accueil (1, 2), dont la largeur est étagée.

4. Implant de renforcement selon l'une des revendications 1 à 3, **caractérisé en ce que** les vis de fixation (29) possèdent des pointes coniques, de préférence en carbure.

5. Implant de renforcement selon l'une des revendications 1 à 4, **caractérisé en ce que** les trous de fixation (15, 16, 25, 26) sont pourvus de dispositifs de sécurité qui empêchent un détachement involontaire.

6. Implant de renforcement selon l'une des revendications précédentes, **caractérisé en ce que** les manchons d'accueil (1, 2) possèdent une longueur au moins égale au triple de la largeur de la tige d'ancrage (96, 97).

7. Implant de renforcement selon l'une des revendications précédentes, **caractérisé en ce que** la pièce de couplage (3) est courte, c'est-à-dire possède une longueur plus petite que la largeur du manchon d'accueil (1, 2).

8. Implant de renforcement selon l'une des revendications précédentes, **caractérisé en ce que** la pièce de couplage (3) est réalisée sous la forme d'un élément de liaison à clavette.

9. Implant de renforcement selon la revendication 8, **caractérisé en ce que** l'élément de liaison à clavette comporte des clavettes plates (31, 32) disposées en sens inverse.

10. Implant de renforcement selon la revendication 8 ou 9, **caractérisé en ce que** des vis de blocage (39) sont prévues pour l'élément de liaison à clavette.

11. Implant de renforcement selon la revendication 10, **caractérisé en ce que** les vis de blocage (39) sont orientées de telle sorte qu'elles se trouvent alignées avec au moins une rangée de trous de fixation (15, 16) au niveau du manchon d'accueil (1, 2).
